(19) Europäisches Patentamt
European Patent Office
Office européen des brevets

(11) Publication number: 0 272 240 A2

(12) **EUROPEAN PATENT APPLICATION**

(21) Application number: 87870181.2

(22) Date of filing: 16.12.87

(51) Int. Cl.4: C07D 207/44 , C08F 222/00 , C08L 35/00 , C08J 5/00

(30) Priority: 18.12.86 US 944157
18.12.86 US 944158
15.06.87 US 62805

(43) Date of publication of application: 22.06.88 Bulletin 88/25

(84) Designated Contracting States: AT BE CH DE ES FR GB GR IT LI LU NL SE

(71) Applicant: Monsanto Company
Patent Department 800 North Lindbergh Boulevard
St. Louis Missouri 63167(US)

(72) Inventor: Dahms, Ronald Herbert
6 Meadowbrook Road
Springfield Massachusetts 01129(US)

(74) Representative: McLean, Peter et al
Monsanto Europe S.A. Patent Department
Avenue de Tervuren 270-272 Letter Box No 1
B-1150 Brussels(BE)

(54) **Maleimide resins.**

(57) Substantially insoluble bismaleimide compositions consisting essentially of bismaleimides of the formula

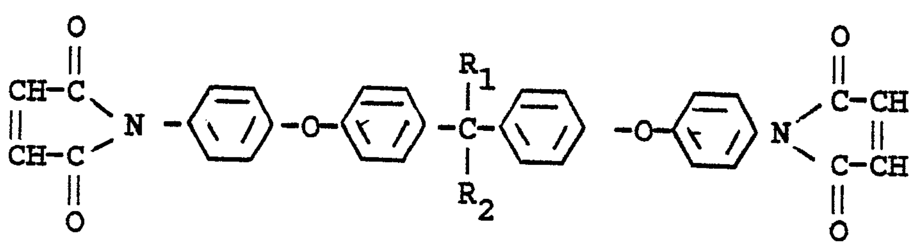

where $R_1$ and $R_2$ are independently hydrogen, methyl, ethyl or halogenated methyl. The phenyl groups may be substituted. The compositions, having a solubility in acetone of less than about 5% by wt., are useful as melt processable resins for high temperature resistant laminates. Mixtures of such bismaleimides and maleamic acids are substantially soluble. Solutions containing polyamines equivalent to amount of maleamic acid are surprisingly stable.

EP 0 272 240 A2

## MALEIMIDE RESINS

Disclosed herein are inventions relating to substantially insoluble aromatic ether bismaleimide resins, methods of providing solutions of such resins, amine-stabilized solutions, and laminates based on such resins.

## BACKGROUND OF THE INVENTION

Bismaleimide resins are advantageously used in providing resin matrix composites, e.g. glass or carbon fiber reinforced laminates, to achieve enhanced properties such as greater thermal stability and lower moisture sensitivity than is possible with other composites, e.g. composites based on epoxy or other resins. A common bismaleimide, i.e. bis(4-maleimidophenyl)methane, exhibits poor solubility in many organic solvents of choice. Its use in commercial manufacture of laminates is facilitated by dissolving the bisimide in N-methyl pyrrolidone (not a preferred solvent) and by chain extension by Michael addition reaction with diamines.

Nishikawa, et al., disclose in U.S. Patent 4,460,783 that certain aromatic ether bismaleimide compounds such as bis(maleimidophenoxyphenyl) propane and the like are highly soluble in desirable solvents such as acetone, toluene, methyl ethyl ketone and the like. See also Harvey et al in "New Aromatic-Ether Bismaleimide Matrix Resins", ANTEC '86, page 1311.

It has been discovered that in their purer forms such aromatic ether bismaleimides have advantageously low solubility for some applications. It has also been discovered that the solubility of such aromatic ether bismaleimides can be enhanced by the presence of a solubilizing amount of maleamic acid group-containing compounds, e.g. precursor having terminal maleamic acid groups not converted to the terminal maleimide group. Such acid-containing compounds advantageously increase the solubility of aromatic ether bismaleimides.

However, since the terminal acid groups will tend to liberate water from ring closing imidization during cure of such resin, such water will be vaporized during normal curing conditions and may tend to generate voids or blisters in fabricated articles such as laminates. For instance, Bargain in U.S. Patent 3,838,358 discloses that bismaleimides, e.g. bis(4-maleimidophenyl)methane, are prepared by reacting a diamine with maleic anhydride followed by catalytic cyclodehydration. The bismaleimide is purified, i.e. separated from acid-containing species, by washing with a basic solution, e.g. aqueous sodium bicarbonate. See also Balme who discloses in U.S. Patent 3,975,401 a process for reducing the proportion of maleimido-acid (i.e. maleamic acid) groups in the precipitated product of cyclodehydration.

Although such acid groups can liberate water during cure conditions, it has been discovered that low amounts of liberated water can be tolerated in many fabrication practices. However, in critical applications it is especially desirable that bisimide resins cure with minimal liberation of water, e.g. to avoid blisters that can form when laminates of the cured bisimide resin are subjected to high temperatures, e.g. as in vapor phase soldering. acid groups will tend to liberate water from ring closing imidization during cure of such resin. Such water will be vaporized during normal curing conditions and may tend to generate voids or blisters in fabricated articles such as laminates.

It has also been discovered that the stability of such solutions of bismaleimides and acid-containing compounds can be enhanced by including polyamine at about the equivalent amount of acid.

## SUMMARY OF THE INVENTION

This invention provides substantially insoluble aromatic ether bismaleimides. This invention also provides soluble mixtures of such maleimides and maleamic acids. Solutions of bismaleimides exhibiting enhanced stability are achieved by providing a minor amount of polyamine, e.g. up to about the equivalent amount of maleamic acid of the composition. Resins that become rapidly viscous upon heating are advantageously provided by utilizing polyamines having secondary amine groups. This invention also provides laminates made from such resins and methods of making such laminates.

DETAILED DESCRIPTION OF THE INVENTION

The substantially insoluble aromatic ether bismaleimide compositions of this invention consist essentially of bismaleimides of the formula

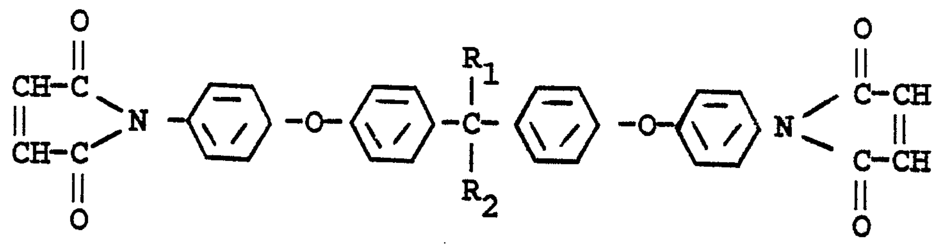

(I)

where $R_1$ and $R_2$ is independently hydrogen, a methyl group, an ethyl group or a halogenated methyl group, e.g. a trifluoro methyl group. The phenyl groups may be unsubstituted or substituted, e.g. with lower alkyl groups, lower alkoxy groups halogens or other common substitutents. A preferred bismaleimide is derived from the diamine 2,2-bis[4-(4-aminophenoxy)phenyl] propane. Such compositions exhibit a solubility in common organic solvents such as acetone of less than about 5 percent by weight at 20°C. To achieve such insolubility the compositions will have only minor amounts of maleamic acid precursor (i.e. the bismaleamic acid and/or the mono maleamic acid precursor) such that the composition exhibits a molar ratio of maleimide groups to maleamic acid groups of greater than 20 to 1. In some applications where minimal water liberation during cure is desired, such compositions may preferably have a molar ratio of maleimide groups to maleamic acid groups of at least about 50 to 1 or higher.

The compositions of this invention are obtained by separating materials consisting essentially of the bismaleimide from the imidization reaction product resulting from ring closing dehydration of maleamic acid precursors. Such cyclodehydration reaction is effected to varying degrees of completion resulting generally in a product comprising a mixture of the bismaleimide, the bismaleamic acid precursor and the half reacted intermediate compound having both maleimide and maleamic acid terminal groups. The preparation of such mixture is disclosed in U.S. Patent 4,460,783. The cyclodehydration of the bismaleamic acid precursor is preferably carried out in the presence of an acid anhydride to assist dehydration, tertiary amine such as triethylamine to assist in ring closing, and a catalyst such as cobalt acetate. Preferably the cyclodehydration is carried out under conditions, readily ascertainable by those skilled in the art, that are sufficiently mild to prevent the formation of substantial amounts of higher molecular weight oligomers.

Purification of the bismaleimide can be achieved by dissolving the imidization reaction product in a solvent such as acetone at levels of up to about 50 percent by weight or higher, say at least about 70 percent by weight. It has been found that the bismaleimide will preferentially precipitate from such solutions. Selective precipitation may occur within a short period of time or may take several hours to commence. Factors influencing the rate and quantity of bismaleimide precipitation are generally known to those skilled in the art and include the initial solids concentration, degree of mixing, degree of cooling and the like. It has generally been found that substantial quantities of the bismaleimide can be separated from a solution upon sitting at room temperature for about 24 hours. Although the bismaleimide is recovered as a precipitate from solution, it is often possible to provide such bismaleimide temporarily in solution, e.g. by vigorous mixing, heating or other known solubilizing techniques. The term "substantially insoluble" as used herein is intended to characterize bismaleimide material that does not form stable solutions. Accordingly, it is a further characteristic of the composition of this invention that when at least about 40 parts of the composition is mixed with acetone at room temperature to provide 100 parts of solution, that substantial quantities, e.g. at least about 25% of said composition is separated from said solution after 24 hours.

Bismaleimide compositions of this invention are substantially insoluble in common organic solvents such as acetone, toluene, methyl ethyl ketone and the like, e.g. exhibit solubilities in acetone not substantially higher than about 5% by weight at room temperature. They do exhibit solubility, however, in aprotic solvents such as N-methyl pyrrolidone, dimethyl acetamide, dimethyl formamide, dimethyl sulfoxide, and the like. Accordingly, the bismaleimide compositions of this invention can be used to provide bismaleimide resin matrix composites by saturating fiber reinforcement materials, such as glass or carbon filament or cloth, woven or non-woven, with a solution of such bismaleimide compounds or with molten bismaleimide compound. Such bismaleimide compounds are cured by heating at temperatures of e.g. 100-300°C.

Although substantially pure compounds of the bismaleimide of the above formula I and the corresponding bismaleamic acid precursor are individually substantially insoluble in common organic solvents such as acetone, methyl ethyl ketone, toluene and the like, it has been surprisingly discovered that highly acetone soluble compositions can be achieved by providing mixtures of such bismaleimides and maleamic acids.

Such highly acetone soluble compositions are soluble in acetone at room temperature at levels of at least 50 percent by weight.

The amount of such maleamic acid-containing compounds is conveniently expressed in terms of "acidity" based on maleamic acid groups as a percent of total acidity when all of the maleimide and maleamic acid groups of the compounds in the mixture are taken as maleamic acid groups. When the mixture is prepared from a known amount of bismaleamic acid precursor, such acidity is readily determined by titrating a sample of the mixture to a neutral endpoint with a standard base, e.g. N/20 KOH; acidity is determined with reference to the titer of the bismaleamic acid precursor.

Preferably, soluble compositions of this invention have an acidity of less than about 20%; more preferably, acidity will be in the range of about 1-15%. In many cases where it is desirable to provide a highly soluble maleimide that liberates low amounts of water upon curing, it is most preferred that acidity be less than about 12%, e.g. about 2-8%. Such soluble compositions can often be achieved by selective control of the cyclodehydration of the maleamic acid-containing precursors.

This invention also provides solutions of highly soluble mixtures of bismaleimides in common organic solvents such as acetone, methyl ethyl ketone and toluene, or mixtures thereof. Such solutions can be provided by solvents selected from the group consisting not only of acetone, methyl ethyl ketone, and toluene but also of methyl isobutyl ketone, tetrahydrofuran, dimethylformamide, dimethylacetamide, dimethylsulfoxide, N-methyl pyrrolidone, ethylene dichloride, and xylene and the like or a mixture of such solvents. In many cases, the solvent of choice will consist essentially of acetone.

Solutions of bismaleimide resin of this invention are useful in providing matrix composites and often such solutions desirably comprise from about 40 to about 90 percent solids of resin mixtures of bismaleimide and maleamic acid. Useful resin solutions will generally have a viscosity between 50 and 500 centipoise, although process requirements may require viscosities outside of that range. In many cases it is especially desirable that such solutions have a viscosity between about 100 and 200 centipoise.

When solutions are intended to be used within a short period of time (e.g. within minutes or even hours, of dissolution) solutions of such mixtures, of at least about 50% by weight or higher, e.g. about 70%, can be obtained. However, in many cases, especially with relatively high concentrations of such mixtures, e.g. at least about 50% by weight, the bismaleimide component of the solution tends to separate over time from the solution in substantial quantities resulting in a solution containing dispro portionately higher amounts of resin having maleamic acid terminal groups. Such maleamic acid terminal groups often undergo ring closing during cure with the result of liberation of water which may undesirably tend to form voids or blisters in composites.

It has been discovered that separation of bismaleimide compounds from solutions can be avoided by providing solutions of such mixtures of maleimides and maleamic acids with a minor amount of a species capable of interacting with maleamic acid groups to form amides, esters, etc., or with terminal unsaturation. Such species can comprise amines, e.g. polyamines. The amount of polyamine present in the solution to provide stability can be conveniently expressed in terms of equivalents of amine groups and maleamic acid groups, e.g. conveniently expressed in terms of the ratio of amine groups to maleamic acid groups. When the polyamine is present in solution such that the amount of amine groups is substantially less than the equivalent amount of maleamic acid groups, solubility may be enhanced but for a shorter period of time than when substantially the equivalent amounts are utilized. Moreover, when the polyamine is present such that the amount of amine groups is substantially greater than the equivalent amount of maleamic acid groups, stability will generally tend to be lessened, often with substantially increased viscosity of the solution. Such viscous solutions may tend to gel rapidly on heating, providing undesirable resin composites.

Considerable latitude can often be employed in determining such equivalence. In many cases it is advantageous to provide solutions where the ratio of amine groups to maleamic acid groups is from about 0.5 to about 4. To achieve exceptionally long stability, e.g. for days or weeks or more, without separation, e.g. by precipitation of a maleimide or maleamic acid component of the mixture, and to prevent an undue increase in viscosity, it is generally useful to provide polyamine so that the amount of amine groups is more nearly the equivalent of maleamic acid groups, e.g. where the ratio of amine groups to maleamic acid groups is about 0.8 to 2, and most preferably about 0.9 to 1.5.

The polyamine can comprise a diamine as used in preparing the bismaleimides of this invention, e.g. 2,2-bis[4-(4-aminophenoxy)phenyl] propane. Alternative diamines can be aryl diamines such as methylene dianiline or alkyl diamines, such as diaminopropane, putrescine, cadaverine, hexamethylene diamine, and the like, or triamines such as triaminononane and the like. It has been found that stable solutions that allow

the resin to rapidly become viscous upon heating are achievable when the polyamine contains secondary amines, such as diethylenetriamine, triethylenetetramine, tetraethylenepentamine, pentaethylenehexamine, and polyethyleneimine. Especially desirable resins that become rapidly viscous upon heating are provided with polyamines containing at least two secondary amines.

In one method of forming resin matrix composites, cloth-like layers such as glass or carbon fiber cloth (woven or non-woven) is saturated with a solution of this invention. Such saturation can be conveniently carried out by dipping such cloth into a resin solution. Excess solution can be removed by passing the cloth through squeeze rolls. Solvent is removed in any convenient manner such as by heating the solution-saturated cloth, e.g. in an oven at a temperature often above the boiling point of the solvent. The length of time at elevated temperature is desirably short, e.g. less than about 10 minutes, but will be sufficiently long to remove solvent and promote partial reaction of the resin to a coherent thermoplastic state (often called B-staging) providing a dry (e.g. solventless but often tacky) resin-impregnated cloth, commonly called a "pre-preg". Such resin-impregnated cloth can then be provided in one or more layers which can be thermoformed, e.g. heated in a compressed stack and cured to provide a laminate by heating for an extended period of time, e.g. about an hour or more, at elevated temperatures say between about 150 and 300°C, preferably at least about 180°C to about 250°C.

An effective amount of polyamine will also facilitate formation of dry resin-impregnated cloth. With low levels of polyamine, the resin will often remain molten, e.g. at temperatures of about 200°C, for undesirably long times, e.g. 20 minutes or more, even hours without reacting sufficiently to form a dry thermoplastic resin. When high levels of polyamine are utilized, e.g. substantially higher than about the equivalent amount of maleamic acid, the resin generally tends to rapidly gel upon heating, providing an undesirable foamy, brittle resin. Desirably the polyamine will assist in providing such dry thermoplastic resin in a short time, say less than about 10 minutes, preferably on the order of about 1 to 2 minutes. The time for formation of such dry thermoplastic resin is often correlated with "Dry Rubber Time", a predictive test defined more particularly herein in Example 4.

Some polyamines, e.g. diamines, allow for advantageous Dry Rubber Times, e.g. about 3 minutes or less, for solutions that are maintained for a short period of time, say about a day or so. However, when such solutions are maintained for longer times, e.g. about a week or more, Dry Rubber Times tend to increase to undesirable levels, e.g. about 5 minutes to 20 minutes or more. Advantageously, polyamines having secondary amine groups allow for short Dry Rubber Times even when solutions are maintained for several weeks. Accordingly, preferred aspects of the inventions disclosed herein comprise polyamines having secondary amine groups.

The solutions of this invention may also comprise a variety of other materials that can be useful in providing laminates with desirable properties. Such materials may include fillers, such as silica, thermoplastics and/or reactants having one or more vinyl, epoxy, or cyanate ester groups, as illustrated in U.S. Patent 4,654,407.

The following disclosure is provided to illustrate specific embodiments and aspects of the invention but does not imply any limitation of the scope of the invention.

## EXAMPLE 1

This example serves to illustrate the preparation of substantially insoluble bis-(maleimidophenoxyphenyl) propane, soluble mixture of maleamic acids and bis(maleimidophenoxyphenyl) propane according to this invention.

351 grams of maleic anhydride and 1,012 grams of acetone were heated to reflux temperature (about 63°C) in a 5-liter reaction flask. A solution of 693 grams of 2,2-bis[4-(4-amino-phenoxy)phenyl] propane in 1,350 grams of acetone was metered into the refluxing solution over a period of 40 minutes. The reaction mixture was held at 30 minutes at reflux temperature to provide essentially 100 percent complete conversion to the bismaleamic acid of 2,2-bis[4-(4-amino-phenoxy)phenyl]propane which precipitated as a yellow powder.

The following materials were added to the suspension of bismaleamic acid in refluxing acetone: 495 grams of acetic anhydride, 3.375 grams of nickel acetate tetrahydrate, and 58.5 grams of triethylamine. The suspension was maintained at reflux temperature for about two hours then cooled to 50°C. The resulting clear solution was stirred into cold water yielding a precipitated yellow powder which was washed with water to remove solubles, filtered and dried in an air oven at 60°C to constant weight. Analysis by high pressure liquid chromatography indicated that the powder comprised about 76 percent of the bismaleimide of 2,2-bis[4-(4-amino-phenoxy)phenyl] propane and about 5 percent of the precursor bismaleamic acid; the

balance of the powder is believed to be the half-imidized intermediate having both maleimide and maleamic acid terminal groups. The powder was dissolved in acetone from 40 to about 60 percent by weight to provide solutions according to this invention.

The maleimide maleamic acid mixture was dissolved in acetone at room temperature (75% solids/25% acetone, by weight). After standing overnight (about 16 hours), about 50% of the solids precipitated from the acetone solution. The acetone-insoluble material was washed with acetone and dried. Analysis by high pressure liquid chromatography indicated that the acetone-insoluble material was about 93% bismaleimide; its solubility in acetone, methyl ethyl ketone and a toluene/acetone solution (50/50) was less than 5% by weight.

The acetone-insoluble bismaleimide will remain in a molten state at about 175°C for greater than about 4 hours. Upon heating to about 250°C, the acetone insoluble bismaleimide cures to a solid thermosetting resin.

## EXAMPLE 2

This example serves to illustrate methods of this invention in providing bismaleimides with solubilizing amounts of maleamic acids.

Bismaleimide of 2,2-bis[4-(4-aminophenoxy)phenyl]propane was prepared essentially as in Example 1 except that the nickel acetate tetrahydrate was replaced with 0.32 g per 100 g of the precursor bismaleamic acid of each of the following cyclodehydration catalysts: cobalt acetate tetrahydrate, nickel acetate tetrahydrate, ferrous acetate, cupric acetate monohydrate, calcium acetate monohydrate, zinc acetate dihydrate, chromium triacetate monohydrate and sodium acetate. The results of the following analyses of the resultant yellow powder are reported in Table 1:

(a) the composition as determined by high pressure liquid chromatography (HPLC) in terms of the area ratios of bismaleimide, what is believed to be the half imidized intermediate and residual bismaleamic acid;

(b) the "acidity" as determined by titration and reported as a percent of the total acidity of the precursor bismaleamic acid; and

(c) the room temperature solubility in acetone in weight percent.

The results reported in Table 1 indicate that high solubility of bismaleimides is highly dependent on the amount of solubilizing maleamic acid and that practice of preferred embodiments of this invention can be facilitated by use of a cobalt, nickel or ferrous acetate as a cyclodehydration catalyst in the preparation of bismaleimide.

Table 1

| Catalyst (cation) | Powder Composition(1) (BMI/MIMA/BMA) | Acidity % | Solubility |
|---|---|---|---|
| Co | 78/15/2 | 3 | >70% |
| Ni | 72/14/6 | 6 | >70% |
| Fe | 62/27/6 | 12 | >70% |
| Cu | 32/21/31 | 23 | <40% |
| Ca | 30/28/30 | 35 | <40% |
| Zn | 30/18/33 | 30 | <40% |
| Cr | 27/27/36 | 37 | <40% |
| Na | 43/33/13 | -- | -- |

(1) Reported in terms of HIPLC area ratios of principal components (i.e. bismaleimide (BMI), maleimide-maleamic acid (MIMA), and bismaleamic acid (BMA).

EXAMPLE 3

This example serves to illustrate the stabilizing effect of polyamines on solutions of mixtures of bismaleimides and maleamic acids and the effect of polyamines on curing of such resins as indicated by Dry Rubber Time.

Dry Rubber Time provides an indication of relative cure rate especially for B-staging and, as specified herein, is a measurement of the time for a sample of resin solution to cure to a dry rubbery mass on a uniformly heated surface. More specifically, about a 1 cc sample of resin (dry or solution) is placed on a 200°C, uniformly-heated cure plate (Thermo-Electric Company, Cleveland, Ohio). The solvent rapidly evaporates as the solution is continuously spread with a spatula forming a molten resin. As the resin reacts to form polymer, thin strings can be pulled from the resin mass by the spatula. As the reaction continues, the resin mass forms into a coherent dry rubbery mass from which polymer strings cannot be drawn. The time at which strings are no longer formed is the "Dry Rubber Time". A Dry Rubber Time of "0" indicates almost immediate gelation of the solution.

The Dry Rubber Time for the resin mixture of bismaleimide and maleamic acids prepared in Example 1 was determined to be greater than three hours (about 10,000 seconds). That is, the test was discontinued after three hours when molten resin (at 200°C) failed to form a coherent rubbery mass.

The addition of polyamine to a solution of such mixture provided substantially short Dry Rubber Times. Acetone solutions (about 60% solids) of the resin mixture of bismaleimides and maleamic acids and various polyamines were prepared as indicated in Table 1 by first dissolving the polyamine in acetone, then adding the resin mixture with agitation. The amount of polyamine is indicated by the ratio of amine groups to acid groups. For instance, the mixture of Example 1 was determined by titration to have 0.0362 equivalents of acid groups per 100 grams; and diaminoethane has 0.0333 equivalents of amine groups per gram (determined by dividing the number of amine groups, i.e. "2", by the molecular weight, i.e. "60"). Thus, adding 100 grams of the resin of Example 1 to a solution containing 0.94 grams of diaminoethane provides a solution where the ratio of amine groups to acid groups is determined to be about 0.86.

The results indicated in Table 2 indicate that polyamine can be advantageously added to solutions of

soluble resin mixtures of bismaleimides and maleamic acids to provide exceptionally long stability of such solutions, e.g. up to three weeks and longer. Moreover the results indicate that certain polyamines can provide exceptionally low Dry Rubber Times, e.g. often less than about 5 minutes less, even after three weeks of storage.

TABLE 2

| Polyamine | R* | Dry Rubber Time (seconds) | | | |
|---|---|---|---|---|---|
| | | As made | 1 day | 6 days | 3 weeks |
| Control-No amine | -- | >10,000 | -- | -- | -- |
| 1,2-diaminoethane | 0.86 | -- | 545 | -- | -- |
| 1,3-diaminopropane | 0.85 | -- | 165 | 230 | 480 |
| " | 1.28 | -- | 130 | 200 | 260 |
| 1,6-diaminohexane | 0.86 | 120 | 190 | 230 | 400 |
| " | 1.29 | 120 | 260 | -- | 360 |
| Triaminononane | 0.87 | 110 | 170 | 450 | 540 |
| " | 1.20 | 80 | -- | -- | -- |
| " | 1.73 | 0 | -- | -- | -- |
| Polypropylene oxide diamine[1] | 0.86 | -- | -- | 1400 | -- |
| Methylene dianiline | 0.85 | -- | 170 | -- | -- |
| " | 1.71 | -- | 100 | >1200 | -- |
| " | 3.40 | -- | 70 | >1200 | -- |
| Bis(aminophenoxyphenyl) propane | 0.86 | -- | 130 | >1200 | -- |
| " | 1.72 | 70 | 100 | >1200 | -- |
| Diethylene triamine | 1.05 | 80 | 90 | 225 | 420 |
| " | 1.21 | -- | 65 | -- | -- |
| " | 1.58 | -- | 40 | -- | -- |
| " | 2.10 | -- | 0 | -- | -- |
| Triethylene tetramine | 1.13 | 100 | 100 | 120 | 220 |
| " | 1.20 | -- | 85 | -- | -- |
| " | 2.27 | -- | 0 | -- | -- |
| Tetraethylene pentamine | 1.23 | 70 | 85 | 90 | 100 |
| " | 1.85 | -- | 0 | -- | -- |
| Pentaethylene hexamine | 1.07 | 90 | -- | -- | -- |
| " | 1.14 | 85 | -- | -- | -- |
| " | 1.29 | 80 | 80 | 90 | 100 |
| " | 1.94 | -- | 0 | -- | -- |
| Polyethylene imine[2] | (2.56 wt.%) | -- | 55 | -- | 55 |

[1]Jeffamine™ D230 (Jefferson Chemical Co.)

[2]Corcat™ P-18 (Virginia Chemicals Co.)

*R: ratio of amine groups to acid groups

EXAMPLE 4

This example serves to illustrate the preparation of dry resin-impregnated cloth and cured laminates according to this invention.

65 grams of the resin mixture of bismaleimide and maleamic acids prepared in Example 1 was added to a solution of 0.975 grams of triethylene tetramine in 35 grams of acetone. (The ratio of amine groups to acid groups was about 1.13). The solution was used to saturate glass cloth. The solution saturated cloth was placed in an air oven (180°C) for about 5 minutes resulting in a dry resin-impregnated cloth (about 38 percent by weight resin). A laminate was prepared by placing 8 sheets of resin-impregnated cloth between two sheets of copper having a density of two ounces per square foot and heating in a press for 2 hours at about 180°C and 3450 kPa (500 psi). The laminate was removed from the press and post cured at 220°C for 24 hours.

The laminate was evaluated in accordance with the procedures of MIL-P-13949F and determined to have the following properties:

Water Absorption (23°C, 24 hours) 0.4%
Dielectric Constant (at $10^6$ cps) 3.5
Dissipation Factor (at $10^6$ cps) 0.01
Copper Peel Strength 1.6 kg/cm (9 lb/in)

The laminate was also floated on molten solder (288°C) for more than 10 minutes. The absence of blistering or delamination illustrate exceptional resistance to thermal stress.

EXAMPLE 5

This example series to illustrate preferred embodiments of this invention where the properties of laminates are effected by the level of maleamic acid in mixtures of bismaleimides.

Laminates were prepared essentially as in Example 5 from materials indicated in Table 3. After the post cure at 220°C the laminates were analyzed for visual signs of delamination as indicated by blisters, e.g. caused by steam generated from water liberated from ring closure of maleamic acids. As reported in Table 3, the laminate prepared from the bismaleimide resin having lower levels of maleamic acid had no visible blisters; the laminate prepared from the resin having higher levels of maleamic acid was severely blistered.

Table 3

| Bismaleimide Mixture (BMI/MIMA/BMA) | TETA[(1)] (g) | Blisters |
|---|---|---|
| 80/12/4 | 0.975 g | None |
| 41/34/10 | 1.65 g | Severe |

(1) TETA: triethylene tetramine

While specific embodiments of the invention have been described, it should be apparent to those skilled in the art that various modifications thereof can be made without departing from the true spirit and scope of the invention. Accordingly, it is intended that the following claims cover all such modifications within the inventive concept.

## Claims

1. A substantially insoluble bismaleimide composition consisting essentially of bismaleimides of the formula

(I)

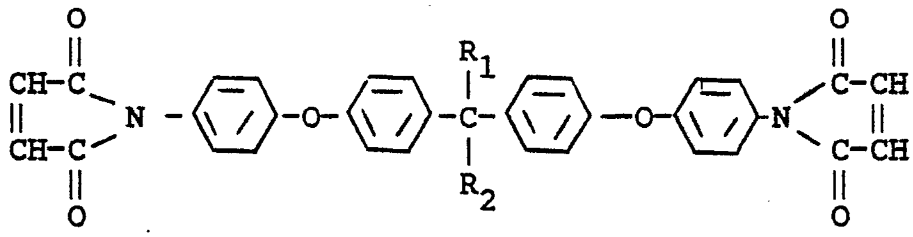

where $R_1$ and $R_2$ is hydrogen, methyl, or ethyl, or halogenated methyl and $R_1$ is the same as $R_2$ or different, having a solubility in acetone at 20°C of less than about 5 percent by weight.

2. The composition of claim 1 comprising minor amounts of amic acid-containing precursor wherein said composition has molar ratio of maleimide groups to precursor maleamic acid groups of greater than about 50 to 1.

3. A substantially soluble composition comprising

(a) bismaleimides of the formula

(I)

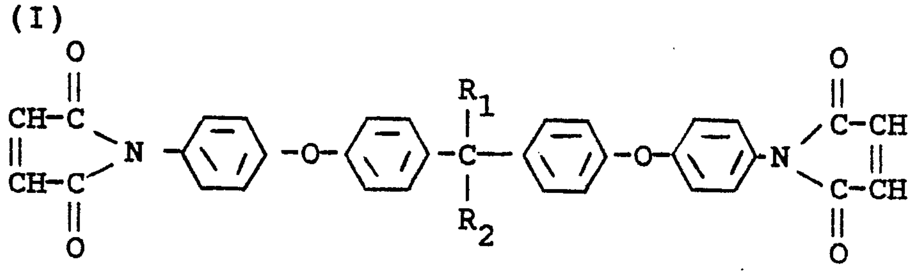

where $R_1$ and $R_2$ is hydrogen, methyl, or ethyl, or halogenated methyl and $R_1$ is the same as $R_2$ or different;

(b) maleamic acids that are precursor of said bismaleimides and

(c) polyamine,

wherein the said maleamic acid precursor is substantially neutralized by said polyamine.

4. A composition according to claim 3 having an acidity determined with reference to the titer of the bismaleamic acid precursor of said bismaleimide and maleamic acids of about 2 to 12%.

5. A composition according to claim 4 wherein the ratio of amine groups of said polyamine to acid groups of said maleamic acids is about 0.9 to 1.5.

6. A composition according to claim 4 wherein said acidity is 3 to 8%.

7. An acetone solution comprising at least 40 percent by weight of the composition according to claim 6.

8. A method of preparing a laiminate comprising:

(a) saturating a plurality of cloth-like layers with a solution according to claim 7 to provide saturated layers;

(b) removing solvent from said saturated layers to provide resin-impregnated layers, and

(c) forming a cured laminate by heating a compressed stack of resin-impregnated layers.

9. A molten solder-resistant laminate comprising a plurality of bismaleimide resin-impregnated, cloth-like layers wherein said resin comprises a composition according to any of claims 1 to 6.